# EUROPEAN PATENT APPLICATION

(11) **EP 4 736 763 A1**
(43) Date of publication of application: **06.05.2026**
(21) Application number: 24306827.7
(22) Date of filing: 30.10.2024
(51) Int. Cl.: A61B 5/16, A61B 5/00

(54) **SYSTEM AND METHOD FOR THE DETECTION AND PREVENTION OF SENSORY ISSUES**

(71) Applicant: Origenes, 75008 Paris (FR)
(72) Inventor: SEGOVIA MARTINE, Manuel, 06330 Roquefort les Pins (FR); LOPEZ, Alejandro, Dublin (IE); KAYZER, Hendrik, Wiefelstede (DE)
(74) Representative: Ipsilon

(57) **Abstract**

The invention relates to a system for detecting and preventing sensory issues in a user, especially a user with a sensory processing disorder, the system comprising at least one wearable sensor and a control unit, said control unit having access to a sensory phenotype of the user, said at least one sensor providing at least environmental information to said control unit and/or said control unit having access to a mental state model of the user,
system wherein, based at least on said sensory phenotype and the mental model of the user and/or the environmental information provided by the sensor, the control unit is configured to compute the probability of a sensory issue occurring for the user and to at least send a detection warning in the case of said probability being above a predefined value.

## Description

### Technical field

The present invention relates to a system and a method for detecting and preventing sensory issues in a user, especially a user with a sensory processing disorder.

### Prior art

Sensory processing disorder (SPD), also known as sensory integration dysfunction, is a condition where multisensory integration is not adequately processed in order to provide appropriate responses to the demands of the environment. Sensory processing disorder is present in almost all people with autism spectrum disorders.

Individuals with SPD can display episodic sensory issues, such as meltdowns or sensory processing overflows, which are mental lockdowns caused by too much sensory input. They are generally triggered by a variety of factors such as stress, social demands, frustration, embarrassment, challenges with communication, emotional triggers, overwhelming aversive sensory stimuli, etc.

The sensory issues related to SPD can be classified as:
- sensory over-responsiveness: the user is reacting beyond expected to a stimulus,
- sensory under-responsiveness: no reaction or little reaction to a stimulus,
- sensory craving: the user looks at a specific stimulus with an exaggerated manner,
- sensory overload or processing overflow: the user's neural system is overwhelmed by the stimuli and exceeds the capability to process and store it, leading to irritation, discomfort and possible meltdown.

These meltdowns are preceded by a pre-meltdown stage, where the individual experiences an increase in agitation, which is also referred to as the rumbling stage. There are several types of interventions that could be implemented in the rumbling stage, wherein such interventions could diminish the meltdowns, both in frequency and severity. For example, removing an individual from a stressful environment, redirecting the individual towards a routine type of activity, moving the individual to a place with a specific structure, can reduce the meltdown intensity or eliminate it altogether. However, these conventional interventions require a caretaker to perform, and are effective only if the caretaker recognizes the rumbling stage. The rumbling stage can be characterized by behaviors which can vary greatly from one individual to another, and at times may appear to be minor such as nail biting, tensing muscles, or otherwise indicating discomfort; all of which can be inadvertently missed by a caretaker. Thus, there is an ongoing need to develop methods for recognizing pre-meltdown behaviors, as well as methods of intervening in pre-meltdown behaviors in order to decrease meltdown incidence and/or severity.

Patent application US 2024/0000342 describes a method for decreasing meltdown incidence and severity in neurodevelopmental disorders. Sensor data is acquired from sensors worn by a subject (motion data, sound data, physiological data) and compared with target data. The method determines if the sensor data is equal to or exceeds the target data. If needed, audible sound therapy is delivered to the subject, with an audio soundtrack familiar to the subject. The audio soundtrack is repeated at least until it is determined that the target data of the subject exceeds the sensor data.

Patent application IN 202121001958 discloses a system for the detection of audio-visual sensory processing disorder, with reduced questionnaire, by using automatic features collected with sensors and facial expressions. The system uses fabric sensors or smart watches to collect body parameters and infrared thermometers to remotely collect temperature. Machine learning is used to analyze the collected data.

Patent application IN 202141019019 discloses a system for assisting a person with an autism spectrum disorder. Thanks to this assistant, they can easily understand other people's emotions and can converse better. The user can also opt for only audio or video options as per their feasibility. The user can also look at an analysis over and over again if they have a hard time understanding the emotions.

International application WO 2020/014780 describes a system for assessing cognitive function that uses tracked electrical activity of the brain of the individuals in response to a specific sequence of stimuli in generating data sets, which, for example, can be encapsulated as a data structure. The data sets can include tracked specific response types, at various times and amplitudes, including, but not limited to, event related potential signal components.

International application WO 2020/203051 discloses a method for estimating a sense that has a high correlation with a mental state of a subject. An acquisition unit acquires information on the activity level of a plurality of sensory areas of the brain of a subject. An estimation unit estimates a sense having a high correlation with a mental state of the subject from among a plurality of types of senses corresponding respectively to the plurality of sensory areas, on the basis of the information of the activity level of each of the plurality of sensory areas acquired by the acquisition unit when the subject is subjected to a plurality of types of stimulation.

There is a need for a method to efficiently warn the user about the probability of a sensory issue in real time and either to allow the user to act on it quite quickly or to provide manual or automatic control of the sensory input to reduce the probability of a sensory issue.

The present invention notably seeks to meet this need.

### Disclosure of the invention

One subject of the invention is a system for detecting and preventing sensory issues in a user, especially a user with a sensory processing disorder, the system comprising at least one wearable sensor and a control unit, said control unit having access to a sensory phenotype of the user, said at least one sensor providing at least environmental information to said control unit and/or said control unit having access to a mental state model of the user, system wherein, based at least on said sensory phenotype and the mental model of the user and/or the environmental information provided by the sensor, the control unit is configured to compute the probability of a sensory issue occurring for the user and to at least send a detection warning in the case of said probability being above a predefined value.

The sensory issues may be audio-visual, tactile or proprioceptive.

The invention links the mental model with the user phenotype to better predict sensory issues, especially related to SPD, and to reduce meltdowns. It uses a framework of individual sensory traits of the user that allows to interpret the mental model.

The system according to the invention allows predicting issues before they are observed on the users. The combination of wearability, real-time analysis, prediction and diagnosis is unique compared to the known methods.

The claimed system also includes the ability to manipulate sensory inputs with the purpose of reducing the probability of experiencing sensory issues, which is not disclosed in the state-of-the-art.

The invention offers an efficient real-time mental state model that understands the condition of the user, especially an SPD user. The mental state model is connected with the environmental conditions to obtain a probability of experiencing a sensory issue.

The method according to the invention may work continuously to reduce the probability of sensory issues.

The phenotype allows individualizing the prediction with clear knowledge of the user, which is also missing in the known methods.

The device is advantageously configurable according to the needs and characteristics of the user.

By *"sensory phenotype"* of a user, it has to be understood the observable sensory processing characteristics of a specific person that reliably relate to the sensory processing issues of that person, especially a user with a sensory processing disorder. A phenotype model advantageously learns the environmental conditions and mental states that characterize the sensory issues of a user. To do so, the different levels of stimulation may be modulated artificially with controlled sources, for example the specific intensity to be delivered is known, or it may be measured ecologically via sensors, such as microphones, photo-diodes, pressure cells, etc.. The intensity of the sensory stimuli is required to generate accurate information of the tolerance of the user. The outcome of this characterization offers the SPD Phenotype, an individual profile of sensory risks for a given user to develop a sensory issue.

The system advantageously comprises a configuration mode, in order to be configured before use.

### Sensors

The system may comprise several wearable environmental sensors, especially head-worn sensors, especially chosen from acoustic sensors and/or microphones, light and/or brightness sensors, cameras, thermometers, or humidity sensors.

The system may further comprise at least one body or mind sensor, especially chosen from sensors pertaining to the activity of the heart, for example electrocardiogram or photo-plethysmography, the activity of the sweat glands during emotional arousal or stress, for example electro-dermal activity or galvanic skin response, the body temperature, the body posture, for example via inertial measurement units, and the activity of the brain, for example via electroencephalography or functional near infrared spectroscopy.

The sensor may be head-worn sensors, for example:
- a microphone to capture sound signals, for the intensity of the sound and/or the timbre, at the ear level of the user to capture physical sound signals that allow to measure the sound level at which the user is exposed, and, based on this, to estimate a perceived sound level, that is to say the loudness or timbre of a sound given a diagnostic measure, that may be part of the SPD phenotype; the sound signals may be used as input for classification of the type of the sound and the identification of critical sound events given the SPD phenotype of the user,
- a light/brightness sensor to measure the level of visual input to the sensory system, in order to detect critical levels given thresholds based on the SPD phenotype of the user,
- a thermometer to capture the environmental temperature in order to detect thermal stress of the body,
- a humidity sensor in order to detect another type of thermal stress of the body,
- a camera for capturing the visual environment in order to classify and detect critical events given the SPD phenotype of the user, especially for assessing the number of people around, that proves to be one of the most important markers that may explain sensory issues.

The wearable body and mind sensors may form a combination of sensors placed in one or several wearable frame(s) and that measure biological signals. "Body sensor" refers to biological signals pertaining to the activity of the heart (i.e., electrocardiogram (ECG) or plethysmography (PPG)); the activity of the sweat glands during emotional arousal or stress (i.e., electro-dermal activity (EDA) a.k.a. galvanic skin response (GSR)); the body temperature; body posture via inertial measurement units (IMUs). "Mind sensor" refers to brain activity via electroencephalography (EEG), functional near infrared spectroscopy (fNIRS) or other sensors dedicated to measuring activity of the brain.

These sensors may be arranged in a modular fashion to suit the needs of the user. The sensors may be placed in the same wearable sensor frame that is sitting in a part of the body of the user where all signals can be measured, for example glasses, headphones, headbands, earphones, etc.. Alternatively, the sensors may be separated into two or more sensor frames distributed in various parts of the body for optimal signal acquisition, notably a ring, a wristband, and a headband.

The sensors may be linked together via a communication protocol that allows the synchronization of the data acquisition in a reliable way, notably UDP, LSL, TCP/IP, OSC, ANT+, WiFi, Bluetooth, etc..

The wearable body and mind sensors advantageously provide information about the physiological state of the user needed to estimate their current mental state.

### Mental state model

In a preferred embodiment, the mental state model consists of a scale of several variables, especially arousal, valence, awareness, acute stress, engagement and mental workload, said variables being especially determined by body or mind sensors.

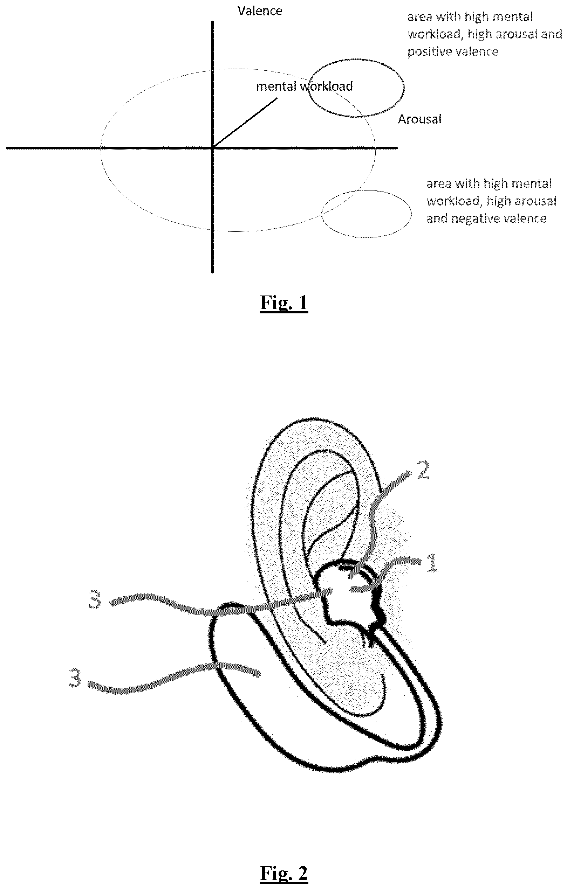

The definition of sensory issue is important for the invention. Having a multidimensional matrix that shows all the mental variables, one may identify the positions where issues can be developed. Figure 1 shows several potential dimensions of the mental model. Arousal, engagement, and awareness are however heavily correlated.

The sensory issues could come from areas that represent a risk for the user. For example, if the model is on high mental workload, high arousal and negative valence then this is a state that if often present would create chronic stress. The evaluation of acute stress may be a specific region of this model. Mental load and negative valence are potential sign of stress.

Other areas that can be declared as sensory issues are not coherent areas. Negative valence and low arousal could identify issues of attention.

The mental state model and the phenotype may be included in an embedded system of the control unit or are remote, especially accessible on the Internet.

### Management modes

The control unit may comprise a user interface to provide information to the user, the user also providing feedback or subjective evaluation of their actual mental state model to the system which then learns from that feedback.

The user interface may be embedded in the wearable frame that comprises the sensors. LEDs may be used to indicate the risk of sensory issues. In a variant, a smart wearable, such as a phone or a watch, may be used. In this case, a graphical user interface may inform on the state of the user with regard to sensory issues. A monitoring station may maintain data logging of the effects of SPD into the user. A professional may then adjust their therapy based on that information. In another embodiment, a remote station may inform a professional of a risk situation related to SPD. For isolated users, this can be a particularly useful feature of the invention.

The system may be configured to send a warning in the case of said probability being above a predefined value to the user, a caretaker or a member of the family of said user.

The sensory phenotype of the user may be characterized by a qualified professional for an initial fitting and/or the sensory phenotype is defined by the user for an initial fitting, the system then self-learning and fine-tuning the fitting as it is used by the user and/or the system being fine-tuned by the user during use.

The characterization of the sensory phenotype may be performed by monitoring the response of the user to several sensory stimuli, especially light, image, sound, taste, smell, touch, and proprioception, at different levels until discomfort or an inability to cope with these stimuli or a combination thereof is registered, said monitoring being performed by using physiological sensors, measuring especially heart rate, skin conductance, pupil dilation, brain activity and by ecological momentary assessment annotations from the user or a qualified professional.

In the case where the computed probability of a sensory issue is above a predefined value, at least one wearable actuator may be available to the system and to the user to manipulate the at least one sensory input responsible for the detected sensory issue.

The system may be configured so that at least one wearable actuator is available to the user in order to manipulate at least one sensory input.

The system may be configured to manipulate automatically at least one predefined stimuli according to the phenotype of the user, using at least one wearable actuator configured to manipulate incoming sensory input by using the required transformation of the sensory input, computed by the control unit.

The system advantageously offers a modular actuator platform that can deliver dynamic stimulation that better suits the need of the user in the presence of a sensory issue or high-risk of a sensory issue. The stimulation may be dynamic and tailored to each circumstance based on information from the environmental context and the SPD phenotype, in order to cover specific modalities, such as touch, hearing, sight, smell and space. This is more efficient than prescribed static therapeutic stimulation modalities, for example sound, music, melody, etc..

The manipulation of said at least one predefined stimuli may depend on the mental state model of the user.

The system advantageously configures the actuators to be efficient for a given user.

The actuator has several action modes to reduce the incidence of the sensory issue, especially amplify, attenuate, transform, modulate, mask, cancel, substitute, block.

The system may be configured to dynamically select actuators that are relevant for a specific sensory issue event. The management system should be capable of analyzing the sensory inputs, and then select the relevant actuators and their configuration in order to be able to transform the sensory input.

The system may be intended to operate in at least five different prediction modes, each requiring a different level in input and system engagement to produce a sensory issue prediction output.

In an embodiment, based on a known SPD phenotype of the user where information about which type(s) of stimuli trigger(s) a sensory issue, these certain types of stimuli are manipulated automatically without consideration of the environmental context nor the mental state of the user. For example, if loud sounds trigger a sensory issue, all loud sounds are attenuated going forward. This may be expressed by: p(sensory issue | phenotype).

In a variant or in combination, based on a known SPD phenotype of the user where information about which particular stimuli trigger a sensory issue, these certain types of stimuli are manipulated considering only the environmental context without consideration of the mental state. For example, if dog barks systematically trigger a sensory issue, all dog barks are automatically suppressed going forward but not all loud sounds. This may be expressed by: p(sensory issue | phenotype, environment).

In a variant or in combination, based on a known SPD phenotype where information about which particular stimuli trigger a sensory issue and how this episode maps onto a mental state of the user, these certain types of stimuli are manipulated considering only the mental state of the user without consideration of the environmental context. For example, stimuli may only be affected if there is an increase in autonomic nervous system activity indicative of distress, regardless of the intensity or presence of the stimuli. If dog barks are known to trigger a sensory issue but at the moment the mental state of the user is robust enough to manage the presence of a dog barking, the dog barks are not suppressed. This may be expressed by: p(sensory issue | phenotype, mental state).

In a variant or in combination, based on a known SPD phenotype where information about which particular stimuli trigger a sensory issue and how this episode map onto a mental state of the user, these certain types of stimuli are manipulated considering both the mental state and the environment. For example, the device constantly monitors both the environmental context and the mental state of the user to decide whether or not to support the user experience. This may be expressed by: p(sensory issue | phenotype, environment, mental state).

In a variant or in combination, regardless of a known SPD phenotype where information about which particular stimuli triggers a sensory issue and how this episode map onto a mental state of the user, the user may control the execution and modality of the stimuli to be manipulated.

The wearable actuators may be chosen from:
- earphones, hearing aids or implantable hearing aids, for manipulating the acoustic input; for example a modified version of the input sound field is played back to the user and/or sound components are added to manipulate the acoustic input,
- glasses or implantable retinal prosthesis where controllable shading, polarization or tinting may be used to manipulate visual input to avoid sensory issues of the visual system by reducing overall brightness or a targeted attenuation of certain spectral components,

- tactile, thermal or electric stimulators, where stimulation may be applied for manipulating the respective sensory input or to mask the user from other modalities,
- pressure controllers for managing pressure points on the joints, which is known to release stress,
- electric gustatory stimulation to act on the smell and/or the taste,
- vestibular implant to act on the balance.

### Method

According to another aspect, the invention also relates to a method for detecting and preventing sensory issues in a user, especially a user with a sensory processing disorder, the method using a system comprising at least one wearable sensor and a control unit, said control unit having access to a phenotype of the user, said at least one sensor providing at least environmental information to said control unit and/or said control unit having access to a mental state model of the user,
the method comprising at least the steps of:
- based at least on said phenotype and the mental model of the user and/or the environmental information provided by the sensor, the control unit computes the probability of a sensory issue occurring for the user, and
- the control unit at least sends a detection warning in the case of said probability being above a predefined value.

Said method according to the invention is advantageously performed by means of computer programs, automatically running on any electronic device comprising a processor, as a mobile phone, a tablet, or a computer, mobile or fixed.

### Computer program product

According to another aspect, the invention also relates to a computer program product for detecting and preventing sensory issues, the computer program product comprising a support and stored on this support instructions that can be read by a processor, these instructions being configured to perform the steps of the method according to the invention.

The features described above in relation with the system apply to the method and to the computer program product.

### Brief description of the drawings

The invention may be better understood upon reading the following detailed description of non-limiting implementation examples thereof and upon studying the appended drawing, in which:
Figure 1, already described, illustrates several potential dimensions of a mental model of a user,
Figure 2 illustrates a wearable sensor used according to the invention, in the form of an ear-worn acoustic device,
Figure 3 illustrates a wearable sensor used according to the invention, in the form of a head-worn visual device,
Figure 4 illustrates a wearable sensor used according to the invention, in the form of a head-worndevice,
Figure 5 illustrates wearable sensors used according to the invention, in the form of integrated glasses and headphones, and
Figure 6 illustrates distributed wearable sensors used according to the invention.

### Detailed description

Preferably and as described above, the system according to the invention aims at detecting sensory issues and predicting their probability of occurrence in a user with a sensory processing disorder. The system comprises at least one wearable sensor and a control unit having access to a sensory phenotype of the user. The sensor provides at least environmental information to the control unit and/or the control unit has access to a mental state model of the user. Based at least on the sensory phenotype and the mental model of the user and/or the environmental information provided by the sensor, the control unit is configured to compute the probability of a sensory issue occurring for the user and to at least send a detection warning in the case of said probability being above a predefined value.

In the illustrated examples and preferably, the system comprises head-worn or ear-worn sensors, an actuator device and a user interface, such as a smartphone or a tablet, haptics, or a mean for visual or acoustic signaling.

Figure 2 shows an example of the system according to the invention in the form of an ear-worn acoustic device. The earphones 1 are used for transmitting playback of modified sound signals. The microphone 2 close to the ear entrance captures sound signals. Around-the-ear or in-ear electrodes 3 capture EEG and other electrophysiological information. In this example, signals are analyzed in the control unit running the mental model and acoustic classification. Based on the results of the analysis, sound signals are advantageously modified, for example certain components are amplified, suppressed, removed, attenuated or masked. The resulting signals are then played back to the user over the earphones.

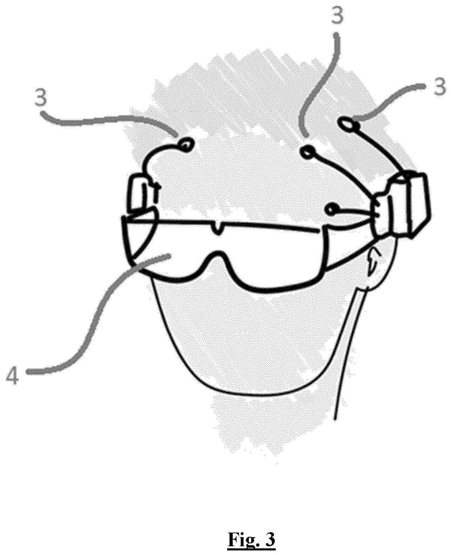

Figure 3 shows an example of the system according to the invention in the form of a head-worn visual device, namely glasses 4 with integrated electrophysiological sensors 3 which capture signals to be analyzed. In this example, images are analyzed in the control unit running the mental model and visual classification. Based on the results of the analysis, vision is advantageously modified, for example certain components are amplified, suppressed, removed, attenuated or masked.

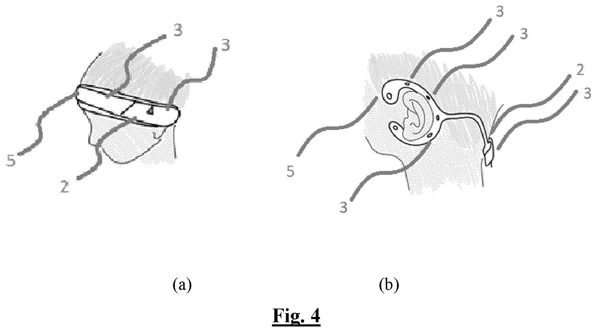

Figure 4 shows an example of the system according to the invention in the form of a head-worn monitoring and alert device, such as a head band, a helmet, a cap or a hat, that contains sensors 3 for capturing input required at least for the mental model. Microphones 2 and brightness sensors 5 are used to capture all required environmental information that is analyzed by the control unit that warns the user of potential sensory issues using a user interface, such as a smartphone or a tablet.

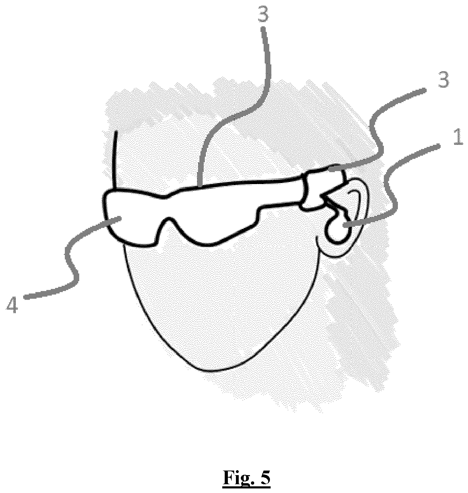

Figure 5 shows an example of the system according to the invention in the form of integrated glasses 4 and earphones 1. Integrated sensors 3 on the device are used to capture electrophysiological signals. If the probability of a sensory issue is detected, both the glasses 4 and/or the earphones 1 can manipulate the sensory inputs.

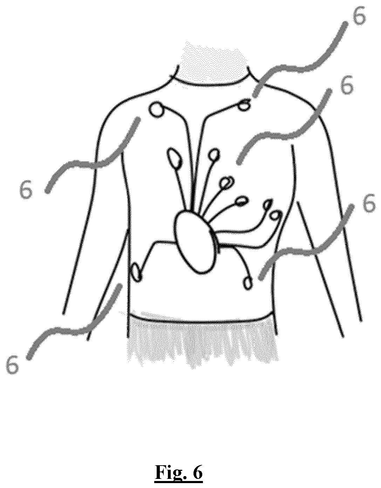

Figure 6 shows an example of the system according to the invention in the form of distributed wearables. Sensors 6 are placed on different part of the head and body to ensure the best sensitivity, being for example attached to clothing, as illustrated, or jewelry.

In a non-illustrated example, the system may comprise a head-worn tactile device, such as glasses or headphones with integrated sensors that capture signals to be analyzed. If tactile modality is needed, the system may deliver a sequence of vibrational stimuli to help the user to manage a sensory issue episode.

Of course, the invention is not limited to the above-described embodiments.

Different sensors or user interfaces may be used.

## Claims

1. System for detecting and preventing sensory issues in a user, especially a user with a sensory processing disorder, the system comprising at least one wearable sensor and a control unit, said control unit having access to a sensory phenotype of the user, said at least one sensor providing at least environmental information to said control unit and/or said control unit having access to a mental state model of the user,
system wherein, based at least on said sensory phenotype and the mental model of the user and/or the environmental information provided by the sensor, the control unit is configured to compute the probability of a sensory issue occurring for the user and to at least send a detection warning in the case of said probability being above a predefined value.

2. System according to claim 1, comprising several wearable environmental sensors, especially head-worn sensors, especially chosen from acoustic sensors and/or microphones, light and/or brightness sensors, cameras, thermometers, or humidity sensors.

3. System according to claim 1 or 2, further comprising at least one body or mind sensor, especially chosen from sensors pertaining to the activity of the heart, for example electrocardiogram or photo-plethysmography, the activity of the sweat glands during emotional arousal or stress, for example electro-dermal activity or galvanic skin response, the body temperature, the body posture, for example via inertial measurement units, and the activity of the brain, for example via electroencephalography or functional near infrared spectroscopy.

4. System according to any of the preceding claims, wherein the mental state model consists of a scale of several variables, especially arousal, valence, awareness, acute stress, engagement and mental workload, said variables being especially determined by body or mind sensors.

5. System according to any of the preceding claims, wherein the control unit comprises a user interface to provide information to the user, the user providing also feedback or subjective evaluation of their actual mental state model to the system which then learns from that feedback.

6. System according to any of the preceding claims, wherein the sensory phenotype of the user is **characterized by** a qualified professional for an initial fitting and/or the sensory phenotype is defined by the user for an initial fitting, the system then self-learning and fine-tuning the fitting as it is used by the user and/or the system being fine-tuned by the user during use.

7. System according to any of the preceding claims, wherein the characterization of the sensory phenotype is performed by monitoring the response of the user to several sensory stimuli, especially light, image, sound, taste, smell, touch, and proprioception, at different levels until discomfort or an inability to cope with these stimuli or a combination thereof is registered, said monitoring being performed by using physiological sensors, measuring especially heart rate, skin conductance, pupil dilation, brain activity and by ecological momentary assessment annotations from the user or a qualified professional.

8. System according to any of the preceding claims, wherein, in the case where the computed probability of a sensory issue is above a predefined value, at least one wearable actuator is available to the system and to the user to manipulate the at least one sensory input responsible for the detected sensory issue.

9. System according to any of the preceding claims, being configured so that at least one wearable actuator is available to the user in order to manipulate at least one sensory input.

10. System according to any of the preceding claims, being configured to manipulate automatically at least one predefined stimuli according to the phenotype of the user, using at least one wearable actuator configured to manipulate incoming sensory input by using the required transformation of the sensory input, computed by the control unit.

11. System according to any one of claims 8 to 10, wherein the actuator has several action modes to reduce the incidence of the sensory issue, especially amplify, attenuate, transform, modulate, mask, cancel, substitute, block.

12. System according to any of the preceding claims, wherein the mental state model and the phenotype are included in an embedded system of the control unit or are remote, especially accessible on the Internet.

13. System according to any of the preceding claims, configured to send a warning in the case of said probability being above a predefined value to the user, a caretaker or a member of the family of said user.

14. Method for detecting and preventing sensory issues in a user, especially a user with a sensory processing disorder, the method using a system comprising at least one wearable sensor and a control unit, said control unit having access to a phenotype of the user, said at least one sensor providing at least environmental information to said control unit and/or said control unit having access to a mental state model of the user,
the method comprising at least the steps of:
- based at least on said phenotype and the mental model of the user and/or the environmental information provided by the sensor, the control unit computes the probability of a sensory issue occurring for the user, and
- the control unit at least sends a detection warning in the case of said probability being above a predefined value.

15. Computer program product for detecting and preventing sensory issues, the computer program product comprising a support and stored on this support instructions that can be read by a processor, these instructions being configured to perform the steps of the method according to the preceding claim.
